# EUROPEAN PATENT APPLICATION

(11) **EP 4 321 169 A1**
(43) Date of publication of application: **14.02.2024**
(21) Application number: 22781662.6
(22) Date of filing: 31.03.2022
(51) Int. Cl.: A61K 38/16, A61P 35/00, A61P 27/02, A61P 29/00, A61P 19/02, A61P 17/06, A23L 33/18, C07K 7/08, C07K 14/71, G01N 33/68

(54) **ANGIOGENESIS INHIBITING PEPTIDE AND USE THEREOF**

(30) Priority: 31.03.2021 KR 20210041705; 30.03.2022 KR 20220039867
(71) Applicant: Seoul National University Hospital, Seoul 03080 (KR)
(72) Inventor: KIM, Hyo-Soo, Seoul 04401 (KR); KWON, Yoo-Wook, Seoul 06088 (KR)
(74) Representative: Nederlandsch Octrooibureau
(86) International application number: PCT/KR2022/004633
(87) International publication number: WO 2022/211543

(57) **Abstract**

The present invention relates to a peptide binding to both a VEGF receptor and a PDGF receptor, a use thereof, and a selection method therefor.

## Description

### [Technical Field]

The present invention relates to an angiogenesis-inhibiting peptide, a use thereof, and the like, and more specifically, to an angiogenesis-inhibiting peptide including any one amino acid sequence selected from the group consisting of SEQ ID NO: 1, SEQ ID NO: 9 and SEQ ID NO: 15, which bind to both a VEGF receptor and a PDGF receptor, a use thereof, and the like.

### [Background Art]

Angiogenesis is a biological process of generating new blood vessels in tissues or organs, and under normal physiological conditions, humans or animals generate new blood vessels only under very limited circumstances. Such angiogenesis occurs through a series of sequential steps including reconstructing vessels and generating new capillaries through decomposition of the vascular basement membrane by proteolytic enzymes, proliferation and migration of vascular endothelial cells that form vessel walls, and the formation of tubes (vasculature) by differentiating the vascular endothelial cells.

Further, the angiogenic process is strictly regulated by various negative and positive regulatory factors, but if such angiogenesis is not normally regulated, it causes pathological disorders such as cancer, diabetic retinopathy, rheumatoid arthritis, inflammation, endometriosis, age-related vision deterioration, psoriasis, and hemangioma.

Diseases associated with angiogenesis may be divided into inflammatory diseases such as arthritis, ophthalmological diseases such as diabetic retinopathy, dermatological diseases such as psoriasis, and cancer (Korean Patent Publication No. 10-2018-0092837). Examples of an ophthalmological disease caused by angiogenesis include diseases such as macular degeneration, diabetic retinopathy as a complication of diabetes, in which capillaries in the retina invade the vitreous body, resulting in blindness, retinopathy of prematurity, and neovascular glaucoma, and millions of people globally lose their eyesight each year due to these diseases. In addition, arthritis is known to be caused by an autoimmune disorder, but chronic inflammation occurring in the synovial cavity induces angiogenesis as arthritis develops, and arthritis is a disease occurring when new capillaries invade joints and cartilage is destroyed. Furthermore, psoriasis is also a chronic proliferative disease occurring on the skin, and since a lot of blood needs to be supplied for rapid proliferation of the disease, angiogenesis cannot help but to actively occur. Further, abnormal angiogenesis serves to supply nutrients and oxygen required for growth and proliferation of tumors, and new capillaries penetrating into tumors provide metastasizing cancer cells with an opportunity to enter the blood circulation system, thereby allowing the cancer cells to metastasize.

Therefore, it is expected that the development of new angiogenesis-inhibiting materials can be effectively used for the prevention, amelioration, treatment, and the like of various diseases induced by or exacerbated by angiogenesis.

### [Disclosure]

### [Technical Problem]

The present invention has been devised to solve the above-described problems in the related art, and the present inventors conducted intensive studies to select a material capable of inhibiting angiogenesis, and as a result, confirmed that a peptide binding to both a VEGF receptor and a PDGF receptor can effectively inhibit angiogenesis, thereby completing the present invention.

An object of the present invention is to provide an angiogenesis-inhibiting composition including a peptide binding to both a VEGF receptor and a PDGF receptor as an active ingredient, a use thereof, and a method for selecting the peptide.

However, the technical problems which the present invention intends to solve are not limited to the technical problems, which have been mentioned above, and other technical problems, which have not been mentioned, will clearly be understood by those with ordinary skill in the art to which the present invention pertains from the following description.

### [Technical Solution]

The present invention provides a composition for inhibiting angiogenesis, including a peptide binding to a vascular endothelial growth factor (VEGF) receptor and a platelet-derived growth factor (PDGF) receptor as an active ingredient.

In an exemplary embodiment of the present invention, the peptide may be characterized by including any one amino acid sequence selected from the group consisting of SEQ ID NO: 1, SEQ ID NO: 9 and SEQ ID NO: 15, and may include a base sequence having a sequence homology of 90% or more, more preferably 95% or more, and most preferably 98% or more with an amino acid sequence of SEQ ID NO: 1, SEQ ID NO: 9, or SEQ ID NO: 15. Since the amino acid sequence represented by SEQ ID NO: 1, SEQ ID NO: 9, or SEQ ID NO: 15 binds to both a vascular endothelial growth factor (VEGF) receptor and a platelet-derived growth factor (PDGF) receptor, a sequence similar to SEQ ID NO: 1, SEQ ID NO: 9, or SEQ ID NO: 15 binds to the VEGF receptor and the PDGF receptor, and thus may exhibit an effect equivalent to the peptide of the present invention, so that the sequence similar to SEQ ID NO: 1, SEQ ID NO: 9, or SEQ ID NO: 15 is not limited. The % sequence homology to an amino acid sequence is confirmed by comparing a comparison region with an optimally aligned sequence, and a portion of the amino acid sequence in the comparison region may include an addition or deletion (that is, gap) compared to a reference sequence (without addition or deletion) for the optimal alignment of the two sequences.

Further, the present invention provides a pharmaceutical composition for preventing or treating an angiogenesis-related disease, including the peptide binding to a vascular endothelial growth factor (VEGF) receptor and a platelet-derived growth factor (PDGF) receptor as an active ingredient.

In addition, the present invention provides a food composition for preventing or ameliorating an angiogenesis-related disease, including the peptide binding to a vascular endothelial growth factor (VEGF) receptor and a platelet-derived growth factor (PDGF) receptor as an active ingredient.

In an exemplary embodiment of the present invention, the angiogenesis-related disease includes all diseases induced by or exacerbated by angiogenesis, and is preferably cancer, diabetic retinopathy, retinopathy of prematurity, neovascular glaucoma, erythema, proliferative retinopathy, wet macular degeneration, psoriasis, hemophilic joints, capillary proliferation in atherosclerotic plaques, keloids, wound granulation, vascular adhesion, rheumatoid arthritis, osteoarthritis, autoimmune diseases, Crohn's disease, restenosis, atherosclerosis, Cat scratch disease, ulcers, glomerulonephritis, diabetic nephropathy, malignant nephrosclerosis, thrombotic microangiopathy, glomerulopathy, pterygia, retrolental fibroplasia, trachoma, and the like, but is not limited thereto.

Furthermore, the present invention provides a method for preventing, ameliorating, or treating an angiogenesis-related disease, the method including administering a composition including the peptide binding to a vascular endothelial growth factor (VEGF) receptor and a platelet-derived growth factor (PDGF) receptor as an active ingredient to a subject in need thereof.

Further, the present invention provides a use of a composition including the peptide binding to a vascular endothelial growth factor (VEGF) receptor and a platelet-derived growth factor (PDGF) receptor as an active ingredient for preventing, ameliorating, or treating an angiogenesis-related disease.

In addition, the present invention provides a use of the peptide binding to a vascular endothelial growth factor (VEGF) receptor and a platelet-derived growth factor (PDGF) receptor for producing a medicine used for an angiogenesis-related disease.

Furthermore, the present invention provides a method for screening a material inhibiting angiogenesis, the method including selecting a material binding to both a vascular endothelial growth factor (VEGF) receptor and a platelet-derived growth factor (PDGF) receptor.

In an exemplary embodiment of the present invention, the material is characterized by binding to any one or more amino acid sequences selected from the group consisting of SEQ ID NOS: 16 to 21.

In another exemplary embodiment of the present invention, the screening of the material is characterized by being performed by a method of measuring the molecular binding of a material binding to any one or more amino acid sequences selected from the group consisting of SEQ ID NOS: 16 to 21, such as surface plasmon resonance (SPR), the BLtz system, fluorescence resonance energy transfer (FRET), and ELISA.

In still another exemplary embodiment of the present invention, the candidate material may be preferably a nucleotide, DNA, RNA, an amino acid, an aptamer, a protein, a compound, a natural product, a natural extract, and the like, but is not limited as long as it is a material capable of binding to a VEGF receptor and a PDGF receptor.

### [Advantageous Effects]

Since the peptide according to the present invention can bind to a VEGF receptor and a PDGF receptor with high binding affinity to inhibit angiogenesis by effectively inhibiting the binding to VEGF and PDGF, it is expected to be applicable to the prevention, amelioration or treatment of all diseases induced by or exacerbated by angiogenesis. Further, it is expected that the new therapeutic agent for angiogenesis-related diseases can be developed by selecting a material commonly binding to a VEGF receptor and a PDGF receptor using the method for screening an angiogenesis inhibiting material according to the present invention.

### [Description of Drawings]

FIG. 1 is a view showing the results of analyzing the binding sequence of a ligand binding to both a VEGF receptor and a PDGF receptor according to an exemplary embodiment of the present invention.

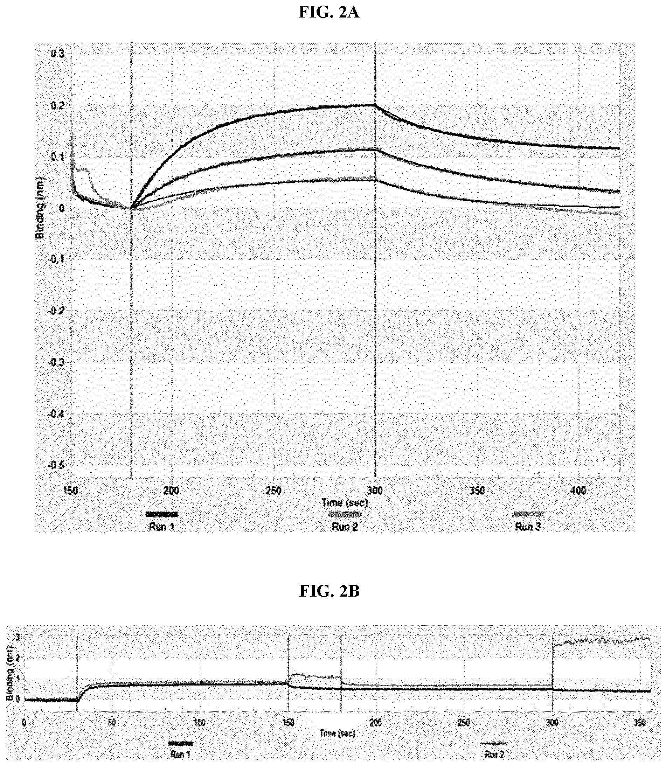

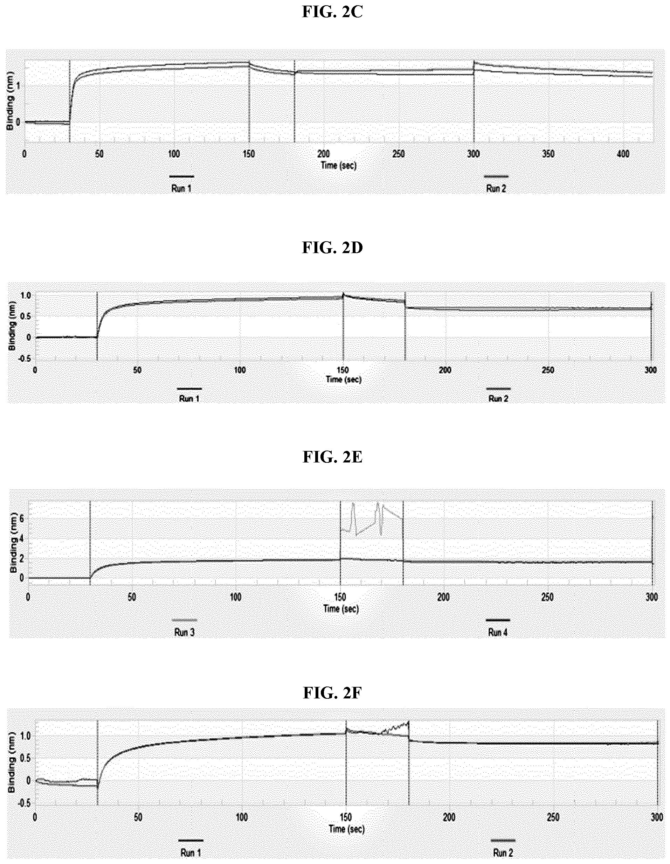

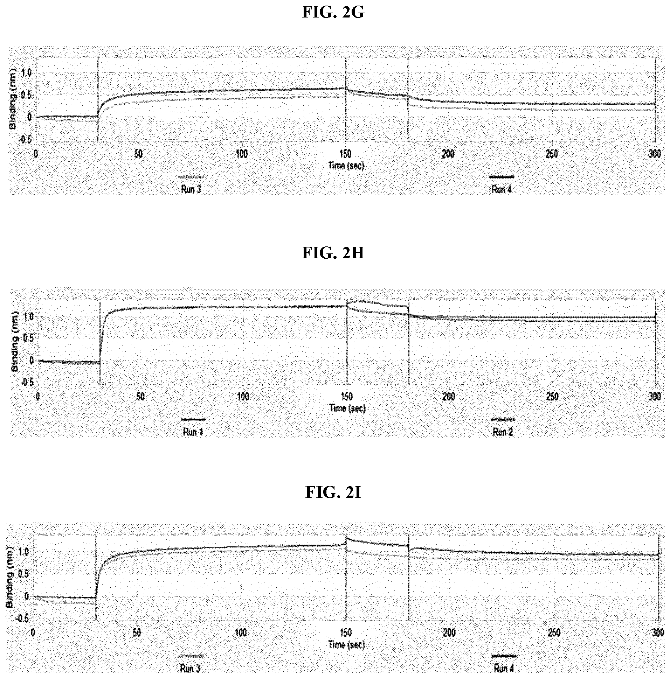

FIG. 2 is a view showing the results of confirming the degree of binding of the peptides using BLtz system according to an exemplary embodiment of the present invention to a VEGF receptor and a PDGF receptor. FIG. 2A is a view showing the results of Peptides 1 to 3, FIG. 2B is a view showing the results of Peptide 8, FIG. 2C is a view showing the results of Peptide 9, FIG. 2D is a view showing the results of Peptide 10, FIG. 2E is a view showing the results of Peptide 11, FIG. 2F is a view showing the results of Peptide 12, FIG. 2G is a view showing the results of Peptide 13, FIG. 2H is a view showing the results of Peptide 14, and FIG. 2I is a view showing the results of Peptide 15.
FIG. 3 is a set of views showing the results of confirming the anticancer effects of the peptide according to an exemplary embodiment of the present invention. * indicates p < 0.05, and ** indicates p < 0.01.
FIG. 4 is a view showing the results of confirming the angiogenesis inhibitory effect of the peptide according to an exemplary embodiment of the present invention in cancer tissue. The white bar indicates 20 µm, * indicates p < 0.05, and ** represents p < 0.01.
FIG. 5 is a view showing the results of observing the angiogenesis inhibitory effect of the peptide according to an exemplary embodiment of the present invention in mice with retinopathy under a fluorescence microscope. The white bar indicates 100 µm.
FIG. 6 is a view showing the results of quantifying the degree of inhibition of angiogenesis of the peptide according to an exemplary embodiment of the present invention in mice with retinopathy. ns indicates non-significant, and * indicates p < 0.05.

### [Modes of the Invention]

The present inventors conducted intensive studies on a method capable of treating angiogenesis-related disease, and as a result, confirmed that angiogenesis can be effectively inhibited using a peptide binding to both a VEGF receptor and a PDGF receptor, thereby completing the present invention. Since a peptide including an amino acid sequence of SEQ ID NO: 1, a peptide including an amino acid sequence of SEQ ID NO: 9, and a peptide including an amino acid sequence of SEQ ID NO: 15 according to the present invention may effectively inhibit angiogenesis by competitively acting with VEGF and PDGF to effectively bind to the VEGF receptor and the PDGF receptor, the peptides may be effectively used for the prevention, amelioration or treatment of diseases induced by or exacerbated by angiogenesis. In addition, by selecting materials specifically binding to sequences such as AXAHTG, QLEXV, IYIXXXD, NNEXS, KDXGXYXCXXR, and EEDXGXYTI, the selected materials may also be used as new agents for preventing, ameliorating, or treating angiogenesis-related diseases.

As used herein, the term angiogenesis refers to a mechanism in which new blood vessels are generated from existing blood vessels, and angiogenesis-related diseases collectively refer to all diseases induced by or exacerbated by abnormal angiogenesis. Angiogenesis-related diseases may refer in particular to diseases associated with the activation of VEGF receptors and/or PDGF receptors, and examples thereof include cancer, diabetic retinopathy, retinopathy of prematurity, neovascular glaucoma, erythema, proliferative retinopathy, wet macular degeneration, psoriasis, hemophilic joints, capillary proliferation in atherosclerotic plaques, keloids, wound granulation, vascular adhesion, rheumatoid arthritis, osteoarthritis, autoimmune diseases, Crohn's disease, restenosis, atherosclerosis, Cat scratch disease, ulcers, glomerulonephritis, diabetic nephropathy, malignant nephrosclerosis, thrombotic microangiopathy, glomerulopathy, pterygia, retrolental fibroplasia, trachoma, and the like, but are not limited thereto.

As used herein, cancer (tumor) collectively refers to a malignant solid tumor that is expected to grow indefinitely along with various hematological cancers that originate from stem cells in hypoxic bone marrow that can expand locally by infiltration and systematically through metastasis. Although not particularly limited thereto, specific examples of cancer include adrenal cancer, bone cancer, brain cancer, breast cancer, bronchial cancer, colon and/or rectal cancer, gallbladder cancer, gastrointestinal cancer, head and neck cancer, kidney cancer, laryngeal cancer, liver cancer, lung cancer, nerve tissue cancer, pancreatic cancer, prostate cancer, parathyroid cancer, skin cancer, stomach cancer, and thyroid cancer. Other examples of cancer include adenocarcinoma, adenoma, basal cell carcinoma, cervical dysplasia and epithelial cancer, Ewing's sarcoma, squamous cell carcinoma, axillary cell carcinoma, malignant brain tumors, blastoma, intestinal ganglion neuroma, hyperplastic corneal nerve cancer, islet cell carcinoma, Kaposi cancer, leiomyoma, leukemia, lymphoma, malignant carcinoid syndrome, malignant melanoma, malignant hypercalcemia, Marpanoid habitus cancer, myeloid cancer, metastatic skin cancer, mucosal neuromas, myelodysplastic syndrome, myeloma, filamentous sarcoma, neuroblastoma, osteosarcoma, osteogenic and other sarcomas, ovarian cancer, pheochromocytoma, polycythemia vera, primary brain tumors, small cell lung cancer, ulcerative and papillary squamous cell carcinoma, seminomas, soft tissue sarcoma, retinoblastoma, rhabdomyoblastoma, renal cell tumors or renal cell carcinoma, reticulum cell sarcoma, and Wilms' tumors. Specific examples of cancer may also include astrocytomas, gastrointestinal stromal tumors (GISTs), gliomas or glioblastomas, renal cell carcinoma (RCC), hepatocellular carcinoma (HCC), pancreatic neuroendocrine cancer, and the like.

As used herein, "prevention" refers to all actions that inhibit an angiogenesis-related disease or delay the onset of the disease by administering the composition according to the present invention.

As used herein, "amelioration" refers to all actions that at least reduce a parameter associated with a condition to be treated, for example, the degree of symptoms. In this case, the composition of the present invention may be used simultaneously with or separately from a drug for the prevention or amelioration of angiogenesis-related diseases.

As used herein, "treatment" refers to all actions in which symptoms of angiogenesis-related diseases are ameliorated or beneficially changed by administering the composition according to the present invention.

As used herein, a subject in need refers to a subject to which the composition of the present invention may be administered, and the subject is not limited.

As used herein, a pharmaceutical composition may be in the form of a capsule, a tablet, a granule, an injection, an ointment, a powder, or a beverage, and the pharmaceutical composition may be characterized in that it is intended for humans. The pharmaceutical composition of the present invention may include a pharmaceutically acceptable carrier. As the pharmaceutically acceptable carrier, a binder, a lubricant, a disintegrant, an excipient, a solubilizing agent, a dispersing agent, a stabilizer, a suspending agent, a colorant, a flavoring agent, and the like may be used for oral administration, in the case of injection, a buffering agent, a preservative, an analgesic, a solubilizer, an isotonic agent, a stabilizer, and the like may be mixed and used, and in the case of topical administration, a base, an excipient, a lubricant, a preservative, and the like may be used. The formulation of the pharmaceutical composition of the present invention may be variously prepared by mixing the pharmaceutical composition of the present invention with the pharmaceutically acceptable carrier as described above. For example, the formulation may be prepared in the form of a tablet, a troche, a capsule, an elixir, a suspension, a syrup, a wafer, and the like when orally administered, and in the case of injection, the injection may be formulated into unit dosage ampoules or in multiple dosage forms. The pharmaceutical composition of the present invention may also be used by being formulated into other sugar-coated tablets, gels, pills, powders, granules, suppositories, external preparations, solutions, suspensions, sustained-release preparations, slurries, and the like. Meanwhile, as an example of suitable carriers, excipients and diluents for formulation, it is possible to use lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, or the like. Further, the pharmaceutical composition of the present invention may further include a filler, an anticoagulant, a lubricant, a wetting agent, a flavoring agent, an emulsifying agent, an antiseptic, and the like.

The route of administration of the pharmaceutical composition according to the present invention is not limited to the following routes, but oral or parenteral administration is preferred, and for example, oral, intravenous, intramuscular, intraarticular, intrasynovial, intraarterial, intramedullary, intrathecal, intracardiac, percutaneous, intradermal, subcutaneous, intraperitoneal, intranasal, intestinal, topical, sublingual, rectal, intrasternal, intralesional, intracranial administration, and the like are included.

The dosage of the pharmaceutical composition of the present invention may vary depending on various factors including the activity of the specific compound used, age, body weight, general health, sex, diet, time of administration, route of administration, rate of excretion, drug combination and the severity of the particular disease to be prevented or treated, and varies depending on the condition, body weight, and the degree of disease of the patient, the form of drug, the route of administration and duration, but may be appropriately selected by a person skilled in the art, and may be 0.0001 to 500 mg/kg or 0.001 to 500 mg/kg daily. The administration may be carried out once daily, or may be divided into several times. The dosage is not intended to limit the scope of the present invention in any way.

In the present specification, the food composition may be used in various foods, for example, beverages, gums, teas, vitamin complexes, and health supplements, and may be used in the form of a pill, a powder, a granule, an infusum, a tablet, a capsule, or a beverage. The food composition includes a health functional food composition. In this case, the amount of the peptide binding to a VEGF receptor and a PDGF receptor in a food or a beverage may be 0.01 to 30 wt% based on the total food weight in the case of the food composition of the present invention, and in the case of the health beverage composition, the peptide binding to a VEGF receptor and a PDGF receptor may be added at a proportion of 0.02 to 10 g, preferably 0.3 to 1 g, based on 100 mL.

The food composition of the present invention has no particular limitation on the ingredient(s) to be added other than the peptide binding to a VEGF receptor and a PDGF receptor, which is an essential ingredient, and may include typical food additives used in the art, for example, natural carbohydrates, flavorants, flavoring agents, colorants, fillers, stabilizers, various nutrients, vitamins, minerals (electrolytes) and the like. As an example of the natural carbohydrates, typical sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol may be used. As an example of the flavorant, a natural flavorant (thaumatin, stevia extract (for example, rebaudioside A, glycyrrhizin and the like), and a synthetic flavorant (saccharin, aspartame and the like) may be advantageously used. As an example of the flavoring agent, honey, D-mannitol, a maltitol solution, a krill concentrate, and the like may be used. In addition to the above, the food composition of the present invention may contain pectic acid and salts thereof, alginic acid and salts thereof, organic acids, protective colloid thickeners, pH adjusters, stabilizers, preservatives, glycerin, alcohols, carbonating agents used in carbonated drinks, and the like. These ingredients may be used either alone or in combinations thereof. The proportion of these additives is not significantly important, but is generally selected within a range of 0 to 20 parts by weight per 100 parts by weight of the composition of the present invention.

Hereinafter, preferred examples for helping with understanding of the present invention will be suggested. However, the following examples are provided only so that the present invention may be more easily understood, and the content of the present invention is not limited by the following examples.

### [Examples]

### Example 1: Selection of sequence binding to VEGF and PDGF receptors

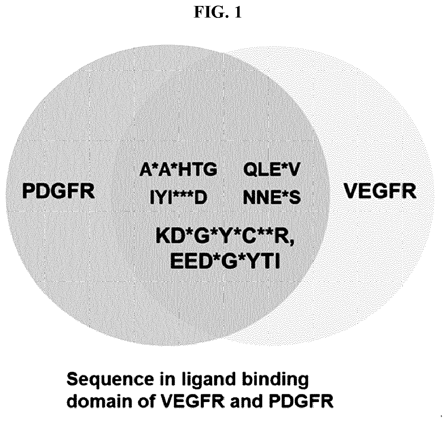

In order to select peptide sequences capable of binding to both a vascular endothelial growth factor (VEGF) receptor and a platelet-derived growth factor (PDGF) receptor, the sequence of a site where both VEGF and PDGF bind in the receptors was analyzed, and a consensus sequence was confirmed using the amino acid sequence analysis program of NCBI BLAST. The results are shown in FIG. 1.

As shown in Figure 1, it was confirmed that AXAHTG (SEQ ID NO: 16), QLEXV (SEQ ID NO: 17), IYIXXXD (SEQ ID NO: 18), NNEXS (SEQ ID NO: 19), KDXGXYXCXXR (SEQ ID NO: 20), and EEDXGXYTI (SEQ ID NO: 21) are base sequences of a site where both VEGF and PDGF bind. An amino acid sequence predicted to be able to bind to the consensus sequence was identified, and peptides including the amino acid sequence were prepared by commissioning AbFrontier.

**[Table 1]**

| Peptide | Amino acid sequence | SEQ ID NO: |
|---|---|---|
| 1 | NRPEVTYPCSCEVKGEEDNS | 1 |
| 2 | NRPEVTAPASCEVKGEEDNS | 2 |
| 3 | NRPEVTYPCSCEVKGAAANS | 3 |
| 4 | NRPEVTAPASCEVKGAAANS | 4 |
| 5 | AAAAAAYPCAAAAAAEEDAA | 5 |
| 6 | AAAAAAAAAAAAAAAEEDAA | 6 |
| 7 | AAAAAAYPCAAAAAAAAAAA | 7 |
| 8 | VKCCGWVSFYNWTDN | 8 |
| 9 | SFYNWTDNAELMN | 9 |
| 10 | AELMNRPEVTYPCSCE | 10 |
| 11 | YPCSCEVKGEEDNSLSVRKGF | 11 |
| 12 | GFCEAPGNRTQSGNHPEDWPVYQEGC | 12 |
| 13 | YPCSCEVKGEEDNSLSV | 13 |
| 14 | RKGFCEAPGNRTQSG | 14 |
| 15 | NHPEDWPVYQEGC | 15 |

It was confirmed that the prepared peptides have binding affinity to VEGF receptors and PDGF receptors using surface plasmon resonance (SPR). More specifically, SPR was performed using a Biacore T200 (GE Healthcare) device and a CM5 chip (GE Healthcare, Cat#. BR-1005-30), and the obtained results were analyzed using BIAevaluation software version 3.0 (GE Healthcare). For a binding test, a concentration of 200 ng/mL was used for the VEGF receptor and a concentration of 400 ng/mL was used for the PDGF receptor. As a result, it was confirmed that Peptide 1, Peptide 9 and Peptide 15 had bound strongly to the VEGF receptor and the PDGF receptor, while Peptide 2 had bound weakly to the receptors. It was confirmed that the remaining peptides did not bind to the receptors.

In addition, the degree of binding between the peptides and the receptors was confirmed in real time using a BLtz instrument (ForteBio). The results are shown in FIGS. 2A to 2I.

As shown in FIG. 2A, it was confirmed that Peptide 1 (green line) showed the highest binding affinity to both the VEGF receptor and the PDGF receptor, and Peptide 2 (pink line) and Peptide 3 (yellow line) had weak binding affinity to the receptors compared to Peptide 1. Further, as shown in FIGS. 2B to 2I, it was confirmed that both Peptide 9 and Peptide 15 had stably bound to the VEGF receptor and the PDGF receptor, and the remaining peptides did not bind to the VEGF receptor and the PDGF receptor, or were not stable.

### Example 2: Confirmation of anticancer effects of peptides binding to VEGF and PDGF receptors

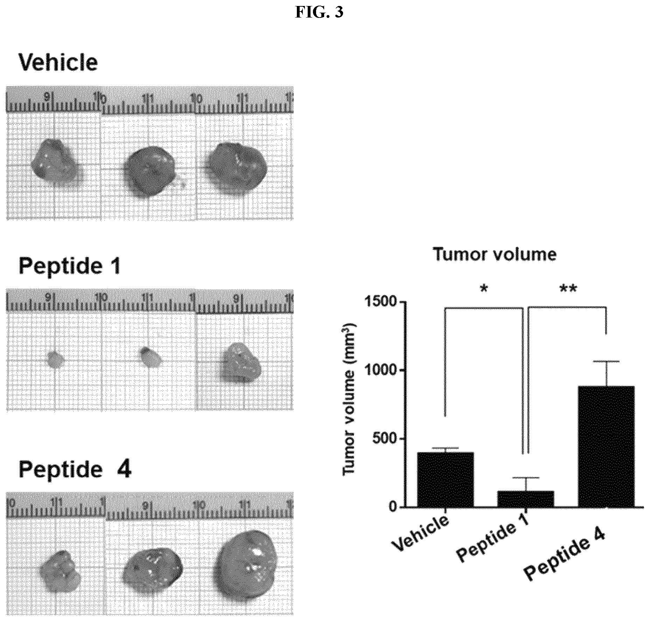

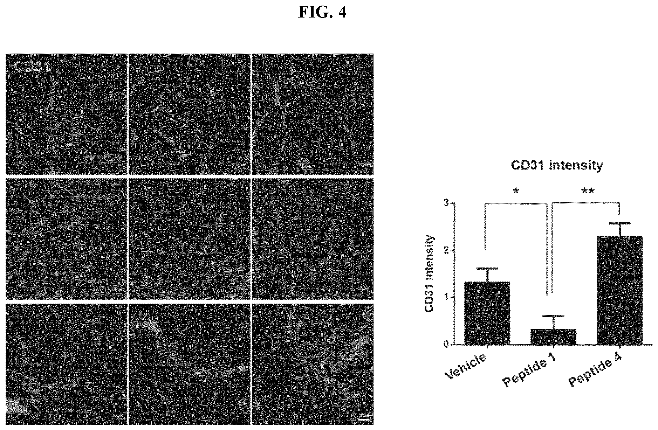

To confirm whether peptides binding to both the VEGF receptor and the PDGF receptor exhibit anticancer effects, 2 X 10⁶ cells of a MDA-MB-231 breast cancer cell line and Peptide 1 (a peptide binding to both the VEGF receptor and the PDGF receptor) or Peptide 4 (a peptide binding to neither the VEGF receptor nor the PDFG receptor), and 100 µL of Matrigel (Corning) were mixed and injected into the subcutaneous tissue of the backs of NSG mice, respectively. As a control, an equal amount of phosphate buffered saline (PBS) was mixed with Matrigel and injected. After the mice were euthanized 14 days later, cancer tissue was obtained and the size of cancer was measured. All animal handling and procedures were performed in accordance with research protocols approved by the Animal Care and Use Committee of Seoul National University Hospital. Thereafter, in all experiments, statistical significance between two groups was confirmed by a Student's t-test, and when P < 0.05, it was determined to be statistically significant. The results are shown in FIG. 3. Then, the obtained cancer tissue was fixed using a 4% paraformaldehyde solution, embedded using paraffin, and then cut to a thickness of 0.4 µm to prepare a tissue section. The prepared tissue section was then stained using a CD31 antibody (Abcam) that specifically binds to CD31, a blood vessel-specific gene, and DAPI. The results are shown in FIG. 4.

As shown in FIG. 3, it was confirmed that when a mixture of Peptide 1 and the cancer cell line was injected, the size of cancer was inhibited compared to the control, but when Peptide 4 and the cancer cell line were mixed and injected, the size of cancer was remarkably increased.

In addition, as shown in FIG. 4, it was confirmed that tissue into which Peptide 1 was injected showed almost no portion stained with a CD31 antibody (red fluorescence), whereas in the control or tissue into which Peptide 4 was injected, the fluorescence intensity for CD31, a blood vessel-specific gene, was remarkably high.

Through the above results, it could be confirmed that Peptide 1 inhibited angiogenesis in cancer tissue, thereby effectively inhibiting the growth of cancer tissue.

### Example 3: Confirmation of therapeutic effect of peptides binding to VEGF and PDGF receptors on diabetic retinopathy

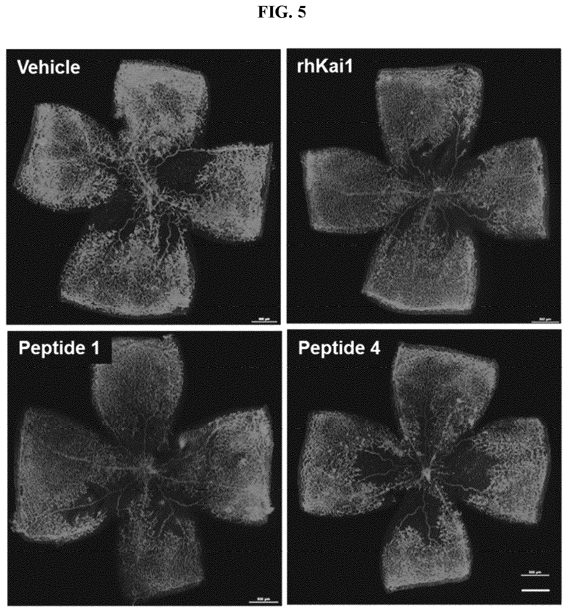

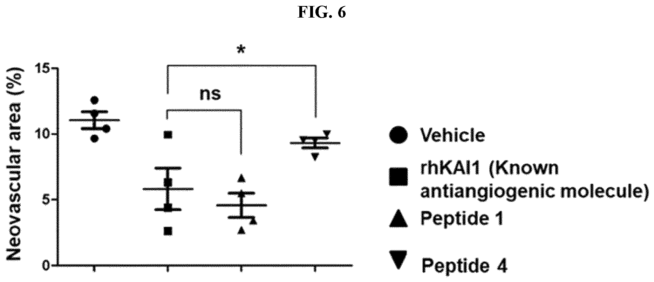

In order to confirm whether a peptide binding to both a VEGF receptor and a PDGF receptor exhibits therapeutic effects on diabetic retinopathy, neonatal mice on day 7 after birth were exposed to a hyperoxia condition (75 ± 0.5% O₂) for 5 days and then transferred to normal atmospheric conditions to prepare mice with oxygen-induced retinopathy. Two days later, for the prepared mice with retinopathy, 1 µL of phosphate buffer solution, Peptide 1, Peptide 4, and KAI1 protein (CD82), which is known to bind to VEGF and PDGF, were intravitreally injected to the right eye of each of the mice, respectively. Mice were euthanized 5 days after injection and enucleated eyes were prepared. The prepared enucleated eye was immunostained using 5 µg/mL Alexa Fluor 594 isolectin GS-IB4 conjugate (Invitrogen), and then observed using a fluorescence microscope. The results are shown in FIG. 5. Thereafter, the area of neovascular tufts was quantified as a percentage of the total retina using Image J (NIH). The results are shown in FIG. 6.

As shown in FIGS. 5 and 6, it was confirmed that the experimental group administered Peptide 1 exhibited an angiogenesis inhibitory effect similar to that of the comparative group administered the KAI1 protein (rhKai1), whereas the experimental group administered Peptide 4 exhibited an angiogenesis inhibitory effect similar to that of the control administered the phosphate buffer solution, and thus did not show an angiogenesis inhibitory effect in retinopathy.

Through the above results, it was confirmed that the peptide including the amino acid sequence of SEQ ID NO: 1, the peptide including the amino acid sequence of SEQ ID NO: 9, and the peptide including the amino acid sequence of SEQ ID NO: 15 according to the present invention can bind to both the VEGF receptor and the PDGF receptor, and it could be confirmed that the peptides effectively inhibit angiogenesis by binding to both the VEGF receptor and the PDGF receptor. Through this, it could be confirmed that the peptide including the amino acid sequence of SEQ ID NO: 1, the peptide including the amino acid sequence of SEQ ID NO: 9, and the peptide including the amino acid sequence of SEQ ID NO: 15 can be used as therapeutic agents for angiogenesis-related diseases, that is, cancer, diabetic retinopathy, retinopathy of prematurity, neovascular glaucoma, erythema, proliferative retinopathy, wet macular degeneration, psoriasis, hemophilic joints, capillary proliferation in atherosclerotic plaques, keloids, wound granulation, vascular adhesion, rheumatoid arthritis, osteoarthritis, autoimmune diseases, Crohn's disease, restenosis, atherosclerosis, Cat scratch disease, ulcers, glomerulonephritis, diabetic nephropathy, malignant nephrosclerosis, thrombotic microangiopathy, glomerulopathy, and the like. Furthermore, it could be confirmed that, by selecting peptides that simultaneously bind to the VEGF receptor and the PDGF receptor, they could be used to develop therapeutic agents for angiogenesis-related diseases.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

Since the peptide according to the present invention can inhibit angiogenesis by effectively inhibiting the binding to VEGF and PDGF, the peptide can be applied to the prevention, amelioration or treatment of all diseases induced by or exacerbated by angiogenesis, for example, cancer, diabetic retinopathy, retinopathy of prematurity, neovascular glaucoma, erythema, proliferative retinopathy, wet macular degeneration, psoriasis, hemophilic joints, capillary proliferation in atherosclerotic plaques, keloids, wound granulation, vascular adhesion, rheumatoid arthritis, osteoarthritis, autoimmune diseases, Crohn's disease, restenosis, atherosclerosis, Cat scratch disease, ulcers, glomerulonephritis, diabetic nephropathy, malignant nephrosclerosis, thrombotic microangiopathy, glomerulopathy, pterygia, retrolental fibroplasia, trachoma, and the like.

## Claims

1. A composition for inhibiting angiogenesis, comprising a peptide binding to a vascular endothelial growth factor (VEGF) receptor and a platelet-derived growth factor (PDGF) receptor as an active ingredient.

2. The composition of claim 1, wherein the peptide comprises any one amino acid sequence selected from the group consisting of SEQ ID NO: 1, SEQ ID NO: 9 and SEQ ID NO: 15.

3. A pharmaceutical composition for preventing or treating an angiogenesis-related disease, comprising a peptide binding to a vascular endothelial growth factor (VEGF) receptor and a platelet-derived growth factor (PDGF) receptor as an active ingredient.

4. The pharmaceutical composition of claim 3, wherein the peptide comprises any one amino acid sequence selected from the group consisting of SEQ ID NO: 1, SEQ ID NO: 9 and SEQ ID NO: 15.

5. The pharmaceutical composition of claim 3, wherein the angiogenesis-related disease is any one or more selected from the group consisting of cancer, diabetic retinopathy, retinopathy of prematurity, neovascular glaucoma, erythema, proliferative retinopathy, wet macular degeneration, psoriasis, hemophilic joints, capillary proliferation in atherosclerotic plaques, keloids, wound granulation, vascular adhesion, rheumatoid arthritis, osteoarthritis, autoimmune diseases, Crohn's disease, restenosis, atherosclerosis, Cat scratch disease, ulcers, glomerulonephritis, diabetic nephropathy, malignant nephrosclerosis, thrombotic microangiopathy, glomerulopathy, pterygia, retrolental fibroplasia, and trachoma.

6. A food composition for preventing or ameliorating an angiogenesis-related disease, comprising a peptide binding to a vascular endothelial growth factor (VEGF) receptor and a platelet-derived growth factor (PDGF) receptor as an active ingredient.

7. The food composition of claim 6, wherein the peptide comprises any one amino acid sequence selected from the group consisting of SEQ ID NO: 1, SEQ ID NO: 9 and SEQ ID NO: 15.

8. The food composition of claim 6, wherein the angiogenesis-related disease is any one or more selected from the group consisting of cancer, diabetic retinopathy, retinopathy of prematurity, neovascular glaucoma, erythema, proliferative retinopathy, wet macular degeneration, psoriasis, hemophilic joints, capillary proliferation in atherosclerotic plaques, keloids, wound granulation, vascular adhesion, rheumatoid arthritis, osteoarthritis, autoimmune diseases, Crohn's disease, restenosis, atherosclerosis, Cat scratch disease, ulcers, glomerulonephritis, diabetic nephropathy, malignant nephrosclerosis, thrombotic microangiopathy, glomerulopathy, pterygia, retrolental fibroplasia, and trachoma.

9. A method for screening a material inhibiting angiogenesis, the method comprising selecting a material binding to both a vascular endothelial growth factor (VEGF) receptor and a platelet-derived growth factor (PDGF) receptor.

10. The method of claim 9, wherein the material binds to one or more amino acid sequences selected from the group consisting of SEQ ID NOs: 16 to 21.

11. A method for preventing, ameliorating, or treating an angiogenesis-related disease, the method comprising administering a composition comprising a peptide binding to a vascular endothelial growth factor (VEGF) receptor and a platelet-derived growth factor (PDGF) receptor as an active ingredient to a subject in need thereof.

12. A use of a composition comprising a peptide binding to a vascular endothelial growth factor (VEGF) receptor and a platelet-derived growth factor (PDGF) receptor as an active ingredient for preventing, ameliorating, or treating an angiogenesis-related disease.

13. A use of a peptide binding to a vascular endothelial growth factor (VEGF) receptor and a platelet-derived growth factor (PDGF) receptor for producing a medicine used for an angiogenesis-related disease.
